# EUROPÄISCHE PATENTSCHRIFT

(11) **EP 1 230 935 B1**
(45) Veröffentlichungstag und Bekanntmachung des Hinweises auf die Patenterteilung: **10.08.2005**
(21) Anmeldenummer: 02002754.6
(22) Anmeldetag: 07.02.2002
(51) Int. Cl.: A61K 47/48, A61K 9/00

(54) **Modifizierte Kolloide und deren Verwendung**
Modified colloids and their use
Colloides modifiées et leures utilisations

(30) Priorität: 09.02.2001 DE 10105921
(43) Veröffentlichungstag der Anmeldung: 14.08.2002
(73) Patentinhaber: B. BRAUN MELSUNGEN AG, 34212 Melsungen (DE)
(72) Erfinder: Meier, Bernd H., 64285 Darmstadt (DE); Jankowiak-Meier, Iris, 64285 Darmstadt (DE)
(74) Vertreter: Weber, Thomas, Dr.

(56) Entgegenhaltungen:
- US-A- 5 704 297
- LOBENBERG R ET AL: "Body distribution of azidothymidine bound to hexyl-cyanoacrylate nanoparticles after i.v. injection to rats" JOURNAL OF CONTROLLED RELEASE, ELSEVIER SCIENCE PUBLISHERS B.V. AMSTERDAM, NL, Bd. 50, Nr. 1-3, 2. Januar 1998 (1998-01-02), Seiten 21-30, XP004107634 ISSN: 0168-3659
- MOCANU G ET AL: "MACROMOLECULAR DRUG CONJUGATES. IV.NICOTINIC ACID AND NICOTINAMIDE/DEXTRAN DERIVATIVES" STP PHARMA SCIENCES, PARIS, FR, Bd. 4, Nr. 4, 1994, Seiten 287-291, XP000909810 ISSN: 1157-1489
- MOCANU G ET AL: "Macromolecular drug conjugates. V. Theophylline-dextran" JOURNAL OF CONTROLLED RELEASE, ELSEVIER SCIENCE PUBLISHERS B.V. AMSTERDAM, NL, Bd. 40, Nr. 1, 1. Juni 1996 (1996-06-01), Seiten 1-9, XP004037346 ISSN: 0168-3659
- KREUTER J: "NANOPARTICLE-BASED DRUG DELIVERY SYSTEMS" JOURNAL OF CONTROLLED RELEASE, ELSEVIER SCIENCE PUBLISHERS B.V. AMSTERDAM, NL, Bd. 16, Nr. 1 / 2, 1. Juni 1991 (1991-06-01), Seiten 169-176, XP000219660 ISSN: 0168-3659

## Beschreibung

Die Erfindung betrifft Verbindungen, die erhältlich sind durch kovalente Anknüpfung von Wirkstoffen oder Gruppen, die sich von Kolloiden ableiten an ein kolloid-wirksames Primärmolekül, die Herstellung derartiger modifizierter Kolloide und deren Verwendung.

Die Erfindung betrifft somit an Kolloide gebundene Arzneiwirkstoffe, ein Verfahren zu deren Herstellung, deren Verwendung zur direkten Injektion sowie Zellsuspensionen umfassend diese an Kolloide gebundenen Arzneiwirkstoffe in phagozytierter Form und deren Verwendung für den gezielten Arzneistofftransport an bestimmte Wirkorte des menschlichen und tierischen Körpers. Die Erfindung betrifft ferner ein Kit-of-parts umfassend die Einzelbestandteile zur Herstellung der wässrigen pharmazeutischen Zusammensetzungen.

Ein Arzneistoff, der in den Blutkreislauf des Körpers injiziert worden ist, wird mit dem zirkulierenden Blut in den Organen verteilt. In Abhängigkeit von der chemischen Stabilität des Arzneistoffs, seiner Löslichkeit, seiner Molekülgröße, und seiner Elimination entfaltet nur ein Bruchteil der ursprünglich eingebrachten Menge des Arzneistoffs am Wirkort die gewünscht Wirkung. Sehr oft wird ein großer Teil des Arzneistoffs metabolisiert und/oder eliminiert, bevor er überhaupt am Wirkort über einen hinreichend langen Zeitraum in einer relevanten Wirkkonzentration vorgelegen hat.

Demgegenüber zu stellen ist die gezielte Einführung von Arzneistoffen in spezielle Zellen, die unter dem Begriff "active targeting" zusammengefasst wird. Es ist bekannt, dass Chemotherapeutika gegen viszerale Leishmaniosen (z. B. Doxorubicin) speziell in Makrophagen (dem Wirt für Leishmanien) eingeführt werden, um die Erreger in ihren Wirtszellen abzutöten. Diese Phagozytose durch Makrophagen kann durch die Einbringung des Wirkstoffs in spezielle, mit äußeren Mannoseresten versehenen Liposomen erreicht werden.

Es ist ferner bekannt, dass Granulozyten und Monozyten gezielt in die Region von Entzündungsherden einwandern und dort im Verbund mit anderen Granulozyten Kolonien bilden, also in einer höheren Dichte vorliegen. Diese Reaktion wird durch Chemokine (wie das Makrophagen-chemoaktive Peptid MCP-1) und andere Mediatoren (wie u. a. den Granulozyten-Makrophagen-Kolonie stimulierenden Faktor (GM-CSF)) gesteuert.

Es ist außerdem bekannt, dass Monozyten im Gegensatz zu anderen weißen Blutkörperchen eine hinreichend lange Lebensdauer von mehreren Monaten aufweisen. Als phagozytierendes Zellsystem bezieht sich die Erfindung im wesentlichen auf im Blut zirkulierende Monozyten. Monozyten werden aus der Stammzellreihe im Knochenmark entwickelt. Die Zellen gelangen von dort in die Blutbahn, in der sie für einen Zeitraum von 20 - 30 Stunden zirkulieren. In dieser Phase reifen sie und entwickeln ausgeprägte immunologische Leistungen wie
- Phagozytose für Bakterien, Pilze, Parasiten (und Kolloide),
- Zytotoxizität für Parasiten, Tumorzellen, Viren,
- Sekretorische Leistungen wie
- Arachidonsäureabkömmlinge, Zytokine,
- Komplementkomponenten, insgesamt über 100 verschiedene Produkte, immunoregulatorische Funktionen.

Unter diesen Aufgaben kommt der Phagozytosefunktion eine bedeutende Rolle zu. Viele der zytotoxischen und sekretorischen Funktionen werden durch Phagozytose ausgelöst, wobei einige dieser Funktionen an der Expression von Rezeptoren auf der Zelloberfläche erkannt werden können. Sehr komplexe immunologische Funktionen haben Monozyten bei der spezifischen zellulären Immunabwehr. Hierbei spielt die Interaktion mit dendritischen Zellen zur Antigenprozessisierung mit T-Lymphozyten im Zusammenhang mit dem Haupthistokompatibilitätskomplex (MHC) eine wesentliche Rolle. Durch die Aufnahme in Makrophagen können die gewünschten Substanzen (als arzneilich wirksame Stoffe) direkt in lymphatische Organe eingeschleust und dort beispielsweise als Antigen präsentiert werden.

In der Peripherie des RHS wandern bei Entzündungen im Blut zirkulierende Monozyten verstärkt in den Entzündungsherd ein. Diese zielgerichtete Einwanderung wird durch die im vorigen genannten Mediatoren ausgelöst und unterhalten.

Löbenberg R. et al.: "Body distribution of azidothymidine bound to hexylcyanoacrylate nanoparticles after i.v. injection to rats", Journal of Controlled Release, Elsevier Science Publishers B.V. Amsterdam, NL, Bd. 50, Nr. 1-3, 2. Januar 1998 (1998-01-02), Seiten 21 - 30, XP004107634 ISSN: 0168-3659 beschreibt Nanopartikel, in denen die wirksamen Komponenten an Hexylcyanoacrylat absorbiert sind.

Mocancu G. et al.: "Macromolecular Drug Conjugates. IV. Nicotinic Acid And Nicotinamide/Dextran Derivatives" STP Pharma Sciences, Paris, FR, Bd. 4, Nr. 4, 1994, Seiten 287 - 291, XP000909810 ISSN: 1157-1489 beschreibt die Synthese von makromolekularen Konjugaten von Nicotinsäure und Nicotinamid durch Reaktion der Komponenten mit chloroacetylierten, vernetzten Dextranmikropartikeln.

Mocanu G. et al.: "Macromolecular Drug Conjugates. V. Theophyllinedextran" Journal of Controlled Release, Elsevier Science Publishers B.V. Amsterdam, NL, Bd. 40, Nr. 1, 1. Juni 1996 (1996-06-01), Seiten 1 - 9, XP004037346 ISSN: 0168-3659 beschreibt die Synthese von Theophyllin-Dextran Konjugaten, wobei das Dextrangerüst als Mikropartikel vorliegt.

Kreuter J.: "Nanoparticle-based Drug Delivery Systems" Journal of Controlled Release, Elsevier Science Publishers B.V. Amsterdam, NL, Bd. 16, Nr. 1/2, 1. Juni 1991 (1991-06-01), Seiten 169 - 176, XP000219660 ISSN: 0168-3659 beschreibt die Bildung von Wirkstoff enthaltenden Nanopartikeln durch Polymerisation in Gegenwart des Wirkstoffs oder durch Sorption des Wirkstoffs an das bereits gebildete Polymer. Eine kovalente Bindung des Wirkstoffs an Polymer soll vermieden werden.

Dementsprechend war es eine Aufgabe der Erfindung, eine Verbindung zur Verfügung zu stellen, die einen Arzneimittelwirkstoff enthält und diesen Wirkstoff durch Makrophagen in spezifischen Organen des menschlichen und tierischen Körpers zur Wirkung gebracht werden kann.

Die vorliegende Erfindung dient gemäß dem ersten Aspekt der Anreicherung von arzneilich wirksamen Stoffen in Makrophagen und damit in Zellen und Organen des Retikuloendothelialen Systems. Durch Injektion dieser Makrophagen können ebenfalls Bereiche erreicht werden, die Ziel der Migration von zirkulierenden Makrophagen und weißen Blutkörperchen sind.

Durch die Aufnahme der erfindungsgemäßen Verbindungen I in Makrophagen werden Arzneistoffe in spezifischen Organen eines Patienten zur Wirkung gebracht. Dieser Vorgang wird als "drug targeting to macrophages" bezeichnet. Das setzt voraus, dass der arzneilich wirksame Bestandteil oder Stoff unter der drug targeting Prozedur seine arzneiliche Wirkung behält. Deshalb sollte sich der Arzneistoff idealerweise nach Phagozytose, d.h., Aufnahme in spezifische Vesikel des Makrophagen wieder vom Kolloid lösen, jedoch in den Vesikeln verbleiben, bis die Zellen den gewünschten Zielort erreicht haben. Irreversible Bindungen zwischen Arzneistoff Z und Kolloid X sind deshalb in der Regel nicht sinnvoll.

Für die klinisch befriedigende Anwendung von "drug targeting" Prozeduren mit patienteneigenen Zellen müssen einige Bedingungen erfüllt sein:
1. Das Verfahren sollte einfach und schnell am Patienten ohne Vitalitätsverlust der Zellen (z. B. durch Versand in entsprechende Zentren) durchgeführt werden können.
2. Alle Prozeduren müssen unter sterilen Bedingungen, ohne Verunreinigungen insbesondere Pyrogene durchgeführt werden.
3. Nach Inkubation des entnommenen Patientenblutes mit dem gewünschten Arzneistoff sollte es zu einer befriedigenden Aufnahme oder Phagozytose des arzneilich wirksamen Stoffs in die Makrophagen führen.
4. Nach Phagozytose sollte eine Separation der betreffenden Zellen sowie eine Entfernung von nicht aufgenommenen Substraten erfolgen.
5. Die Phagozytose des Substrats sollte einfach quantifiziert und überprüft werden können.
6. Es sollte eine sofort reinfundierbare Zellsuspension hergestellt werden.
7. Das Schicksal einer reinfundierten Zellsuspension sollte sowohl im peripheren Blut als auch in Gewebeproben und im Urin verfolgt werden können (Eliminationszeiten).
8. Die Vorbereitung des Wirkstoffs zur Phagozytose sollte nicht mit einem Verlust der gewünschten Wirkung einhergehen.

Im Idealfall wird der arzneilich wirksame Stoff nach Phagozytose durch den Makrophagen wieder vom Kolloid abgespalten.

Durch Inkubationsversuche konnte am Blut festgestellt werden, dass die Phagozytosekapazität für Kolloide starken Schwankungen unterliegt. Deshalb müsste für die individuelle Optimierung des Verfahrens die Messung der Aufnahme kolloidal gebundener arzneilich wirksamer Stoffe verlangt werden.

Darüber hinaus konnte beobachtet werden, dass durch Phagozytose eine Vielzahl von Substraten aufgenommen wird. Auftrennungen der Zellfraktionen und Cluster durch Gradientensedimentation mit anderen Kolloiden führten zu einer nur schwer abschätzbaren und unerwünschten Phagozytose der Sedimentationsmedien selbst. Darüber hinaus erschwert die Mischung, Separation, Zentrifugation und Dialyse der Zellen mit verschiedenen Lösungen und Medien eine sterile und pyrogenfreie Probenvorbereitung erheblich.

Die Erfindung basiert insbesondere auf Beobachtungen an Makrophagen, die Kolloide aufgenommen haben. Makrophagen bereiten u.a. Antigene enzymatisch für die Präsentation an spezifische immunkompetente Zellen vor, d.h., die Enzymaktivitäten, Mechanismen der Antigenpräsentation und Transportvorgänge von Makrophagen sind aufeinander abgestimmt. Für die Erfindung relevant ist dabei auf der einen Seite, dass das Enzyminventar von Makrophagen eine sehr hohe Aktivität, z. B. Esterasen, aufweist und auf der anderen Seite kolloidosmotische Wirkungen in solchen makrozytären Zellorganellen bestehen, die Kolloide aufgenommen haben.

Erfindungsgemäß werden die Zellen deshalb inkubiert mit Arzneistoff-Kolloid-Verbindungen der allgemeinen Formel I

Mₙ-X-(L-Z)ₘ (I),

wobei
· X eine kolloidwirksame Verbindung,
· Z ein Arzneimittelwirkstoff,
· L einen Linker, durch den X mit Z kovalent verknüpft ist,
· M ein Fluoreszenzmarker,
· m eine ganze Zahl zwischen 1 und 1.000.000, vorzugsweise 10 und 100.000, besonders bevorzugt 100 und 10.000 und
· n 0 oder 1 ist.

Im Gegensatz zur Verwendung des isolierten Arzneistoffs Z wurden diese erfindungsgemäßen Verbindungen verstärkt mittels Phagozytose durch Makrophagen aufgenommen.

In der Arzneistoff-Kolloid-Verbindung Mₙ-X-(L-Z)ₘ ist der Arzneistoff Z kovalent über eine Linker-Gruppe (L) an die kolloidwirksame Verbindung X geknüpft. Vorzugsweise ist die kovalente chemische Bindung von X an Z über den Linker L reversibel, also ohne weiteres, beispielsweise enzymatisch, wieder spaltbar, wodurch es zur Freisetzung des Arzneiwirkstoffes Z kommt. Geeignete reversible Linker sind vor allem Esterbindungen, die durch die Esteraseaktivität der Makrophagen nach der Phagozytose wieder gespalten werden können. Alternativ können auch Urethan- oder Carbonsäureamid-Gruppen als Linker fungieren. Zur Bildung des Linkers weisen der Arzneistoff Z und das Kolloid X komplementäre, zur Reaktion miteinander geeignete funktionelle Gruppen auf.

Durch eine geeignete Wahl des mittleren Molekulargewichts des Kolloids können die notwendigen Bedingungen für eine Gradientensedimentation deutlich verbessert werden. Gleichzeitig kann die Arzneistoff-Kolloid-Verbindung Mₙ-X-(L-Z)ₘ auch als Träger von Markierungsmerkmalen M dienen, wie beispielsweise Fluoresceinisothiocyanat (FITC), Phycoerythrin, Rhodamid. Bevorzugt findet Fluoresceinisothiocyanat Verwendung.

Ein weiterer Aspekt der Erfindung ist eine Hydrophilisierung von an sich hydrophoben Arzneiwirkstoffen (Z) durch die Bindung an ein hydrophiles Kolloid unter Bildung der erfindungsgemäßen Verbindung Mₙ-X-(L-Z)ₘ entsprechend der obigen Definitionen. Die Herstellung kann wie unten beschrieben erfolgen. Eine solche Wirkstoff-Anbindung an Kolloide ermöglicht die schmerzfreie direkte Injektion von lipophilen Wirkstoffen, wie z. B. Propofol, in wässrigen Lösungen ohne vorhergehende (ex-vivo) Phagozytose.

Erfindungsgemäß werden die Molekulargewichte und Größen so gewählt, dass sie die bei der Reinigung der aufgetrennten Zellsuspensionen durchströmten Dialysemembranen permeieren können. Bei Versuchen konnte festgestellt werden, dass durch dieses Verfahren eine schnelle und befriedigende Trennung der Zellen durch Zentrifugation erfolgen kann und gleichzeitig die Aufnahme der kolloidalen Makromoleküle Mₙ-X-(L-Z)ₘ in die Monocyten und Makrophagen erfolgt.

Ein geeignetes Arzneistoff-Kolloid weist vorzugsweise ein mittleres Molekulargewicht von 20.000 bis 1.200.000 Dalton, bevorzugt 85.000 bis 750.000 Dalton, ganz besonders bevorzugt 100.000 bis 500.000 Dalton auf und liegt in Lösung in einer Konzentration von 0,1 bis 20 Gew.-%, vorzugsweise von 1 bis 15 Gew.-%, besonders bevorzugt von 3 bis 10 Gew.-% vor. Als Elektrolyte können Natrium und Chlorid in einer Konzentration von jeweils 100 bis 154 mmol/l vorliegen. Zur Optimierung der pH-Bedingungen kann die Lösung mit einem Puffer vorteilhaft auf der Basis von Histidinhydrochlorid in gewünschter Weise eingestellt werden. Zusätzlich kann ein Teil der Ionen durch Glucose und/oder Lactat als Nährstoff ersetzt werden.

Dass der Erfindung zugrunde liegende Prinzip nutzt die Aufnahme- und Transportfähigkeit von Makrophagen für Kolloide. Durch chemische Bindung L wird ein arzneilich wirksamer Stoff Z an ein Kolloid X gebunden. In vorteilhafter Ausgestaltung der Erfindung werden die Arzneistoffe Z durch Esterbindungen L an das Kolloid X gebunden. Als Kolloid X kommen hierbei in erster Linie Kohlenhydrate, bevorzugt Polysaccaride wie Stärke oder deren markierte, insbesondere fluoreszenzmarkierte Derivate, vorzugsweise FITC-markierte Stärke, Hydroxyethylstärke, FITC-markierte Hydroxyethylstärke, Dextran, FITC-markiertes Dextran, Carboxymethylstärke (FITC-markierte Carboxymethylstärke) und Amylopektin in Frage. In einer Ausgestaltung kann die Gruppe X ausgewählt sein aus der Gruppe der Hydroxyethylstärken ("HES", "Polyhydroxyethylstärken") mit einem mittleren Molekulargewicht Mw von >100.000, vorzugsweise >150.000 bis zu 500.000, vorzugsweise bis zu 450.000. Der Substitutionsgrad DS beträgt dabei <0,6 und vorzugsweise <0,4.

Alternativ können jedoch auch andere Kolloide umfassend Peptide, z.B. Proteine, Gelatine, Oxypolygelatine, Oligopeptide und Polypeptide und Kombinationen der genannten Kolloide verwendet werden. Geeignete Kolloide und/oder Kolloidgemische weisen ein Molekulargewicht von 20.000 bis 800.000 Dalton, bevorzugt 80.000 bis 600.000 Dalton, besonders bevorzugt 100.000 bis 500.000 Dalton auf.

Vorteilhaft können weitere spezielle Reste in die Kolloide mit eingeführt werden, die eine chemische Einbindung der arzneilich wirksamen Substanz erlauben, zum Beispiel Biotin, Aminosäuren oder Sulfidgruppen tragende Reste, wie Cystein.

Als Arzneistoffe Z kommen alle Substanzen in Frage, die über einen Linker L an die oben genannten Kolloide kovalent eingebunden werden können, also eine funktionelle Gruppe aufweisen, die unter Bildung des Linkers L mit einer komplementären funktionellen Gruppe des Kolloids reagieren können. Besonders bevorzugt sind dabei Arzneiwirkstoffe, die ausgewählt sind aus der Gruppe bestehend aus Antibiotika, Chemotherapeutika, Cytostatika, Antigenen, Oligonucleotiden, Mediatoren, falschen Stoffwechselsubstraten und cytotoxischen Substanzen. Diese Arzneiwirkstoffe werden kovalent an das Kolloid gebunden und makrozytär aufgenommen.

Die als Linker L vorzugsweise fungierende Esterbindung erweist sich dabei in Abhängigkeit von der chemischen Struktur und Polarität der Arzneistoffreste als unterschiedlich stabil. Von herausragender Bedeutung für die Stabilität der Esterbindungen sind die Eigenschaften bzw. das enzymatische Potential der Zellen und Zellorganellen, in die die Arzneistoff-Kolloid-Verbindungen Mₙ-X-(L-Z)ₘ aufgenommen werden. Die Stabilität der durch Phagozytose erreichten Verteilung kann durch die Einführung weiterer auch unterschiedlicher Substituenten verstärkt werden. Dieser Effekt kann auf im wesentlichen zwei gleichsinnig wirkende Mechanismen zurückgeführt werden:
I. Die Esterbindung zwischen Arzneistoff und Kolloid wird chemisch stabilisiert.
II. Durch die in die Zellorganellen und Zellen aufgenommene Kolloide wird ein kolloidosmotischer Druckgradient aufgebaut, der den nach Verseifung freien Arzneistoff in dem betroffenen Kompartiment zurückhält. Durch eine Erhöhung der Substitution wird die enzymatische Degradation beispielsweise eines Stärkemoleküls wesentlich aufgehalten und damit ein höhere kolloidosmotischer Effekt bewahrt.

Beide Mechanismen müssen durch eine höhere Substitution mit esterasestabilen Substituenten entsprechend verstärkt werden.

Im Fall der Verwendung von Carboxymethylgruppen bieten sich weitere Vorteile, weil die in die Esterbindung eingehende Carboxylgruppe von seiten des Kolloids gestellt wird und von dem Arzneistoff lediglich eine Hydroxylgruppe zur Verfügung gestellt werden muss. Zur Modifikation der Phagozytose- und Hydratationseigenschaften kann eine zusätzliche Substitution mit Hydroxyethylgruppen erfolgen.

Neben den Estern stellen Carbonsäureamide und Urethane eine alternative Ausgestaltung des Linkers L zur Anbindung des Arzneiwirkstoffes Z an das Kolloid X dar.

Weist das Kolloid X mehrere funktionelle Gruppen auf (gekennzeichnet durch m>1), so können mehrere Wirkstoffmoleküle Z am Kolloid angebunden werden. Dabei muss es sich keineswegs um identische Wirkstoffmoleküle handeln.

In einer besonderen Ausgestaltung der Erfindung werden unterschiedliche Wirkstoffmoleküle Z₁-Zₙ über unterschiedliche Linker L₁-Lₚ an ein Kolloid X angebunden. Aufgrund unterschiedlicher Hydrolysegeschwindigkeiten k₁-kₚ der einzelnen Linker L₁-Lₚ kann eine zeitverzögerte Freisetzung der einzelnen Wirkstoffe Z₁-Zₚ erfolgen.

Die Bildung des Linkers L kann mit Hilfe der im Stand der Technik beschriebenen Methoden zur Bildung von Carbonsäureestern, -amiden und Urethanen erfolgen.

Ausgehend von einem zugrundegelegten, Hydroxyl-Gruppen (OH-) tragenden Kolloid X, kann die Bildung der Linkers L mit Arzneiwirkstoffen Z erfolgen, die Funktionalitäten besitzen, welche ausgewählt sind aus
· Isocyanat- (-NCO)
· Carboxyl- (-COOH),
· Carbonsäurehalogenid- (-CO-X, mit X = Cl, Br, I)
· Carboxylalkylen- (-(CH₂)_{q}-COOH, mit q = 1-10)
· oder Ester-Gruppen (-COOR).

Umgekehrt können zugrundegelegte, Hydroxyl-Gruppen (OH-) tragende Arzneiwirkstoffe Z, den Linker L mit Kolloiden X bilden, die Funktionalitäten besitzen, welche ausgewählt sind aus
· Isocyanat- (-NCO)
· Carboxyl- (-COOH),
· Carbonsäurehalogenid- (-CO-A, mit A = Cl, Br, I)
· Carboxylalkylen- (-(CH₂)_{q}-COOH, mit q = 1-10)
· oder Ester-Gruppen (-COOR).

Ausgehend von einem zugrundegelegten, Amino-Gruppen (-NH₂) tragenden Kolloid X, kann die Bildung der Linkers L mit Arzneiwirkstoffen Z erfolgen, die Funktionalitäten besitzen, welche ausgewählt sind aus
· Carboxyl- (-COOH),
· Carbonsäurehalogenid- (-CO-A, mit A = Cl, Br, I)
· Carboxylalkylen- (-(CH₂)_{q}-COOH, mit q = 1-10)
· oder Ester-Gruppen (-COOR).

Umgekehrt können zugrundegelegte, Amino-Gruppen (-NH₂) tragende Arzneiwirkstoffe Z den Linker L mit Kolloiden X bilden, die Funktionalitäten besitzen, welche ausgewählt sind aus
· Carboxyl- (-COOH),
· Carbonsäurehalogenid- (-CO-A, mit A = Cl, Br, I)
· Carboxylalkylen- (-(CH₂)_{q}-COOH, mit q = 1-10)
· oder Ester-Gruppen (-COOR).

Die jeweils in den Ester-Gruppen auftretende Gruppe R ist eine Kohlenwasserstoff-Gruppe mit 1 bis 12 Kohlenstoffatomen.

Alternativ ist die erfindungsgemäße Verbindung auch erhältlich durch Reaktion wenigstens einer freien
· Hydroxyl- (-OH) oder
· Amino-Gruppe (-NH₂)
des zugrundeliegenden Kolloids X, mit einer freien
· Carboxyl- (-COOH),
· Carboxylalkylen- (-(CH₂)_{q}-COOH, mit q = 1-10)
· Carbonsäurehalogenid- (-CO-A, mit A = Cl, Br oder I)
· oder Ester-Gruppe (-COOR)
des zugrundeliegenden Arzneimittelwirkstoffes Z, unter Bildung des Linkers L.

In einer weiteren Ausgestaltungsform der Erfindung können zusätzliche, bi-, tri- oder polyfunktionelle Moleküle zur Bildung der Linker eingesetzt werden. Dabei handelt es sich um Verbindungen der allgemeinen Formel

R¹-Yₛ

wobei s eine ganze Zahl von 1 bis 20 ist,
R¹ ausgewählt ist aus
· einer Einfachbindung;
· linearen oder verzweigten, gesättigten oder ungesättigten, aliphatischen oder alicyclischen Kohlenwasserstoffresten mit 1 bis 22, vorzugsweise 2 bis 15, besonders bevorzugt 3 bis 8 Kohlenstoffatomen;
· Aryl-, Aryl-C₁-C₄-Alkyl- und Aryl-C₂-C₆-Alkenylgruppen mit 5 bis 12, vorzugsweise 6 bis 12, besonders bevorzugt 6 Kohlenstoffatomen im Arylrest, die gegebenenfalls substituiert sein können mit C₁-C₆-Alkyl- und/oder C₂-C₆-Alkoxygruppen; oder
· Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
und die Reste Y₁ bis Yₛ funktionellen Gruppen sind, die unabhängig voneinander ausgewählt sind aus
· Hydroxyl- (-OH)
· Amino- (-NH₂)
· Carboxyl- (-COOH),
· Isocyanat- (-NCO),
· Carbonsäurehalogenid- (-CO-A, mit A = Cl, Br oder I)
· Carboxylalkylen- (-(CH₂)_{q}-COOH, mit q = 1-10)
· oder Ester-Gruppen (-COOR).

Bei diesen Verbindungen handelt es sich bevorzugt um trifunktionale Moleküle mit Y₁ = Y₂ = Y₃, besonders bevorzugt jedoch um bifunktionale Moleküle bei denen Y₁ und Y₂ identisch sind.

Solche Verbindungen sind z.B.
· Dicarbonsäuren, wie Oxalsäure, Malonsäure, Bernsteinsäure, Glutarsäure, Adipinsäure, Pimelinsäure, Azelainsäure, Sebacinsäure, Maleinsäure, Fumarsäure, Sorbinsäure, Phthalsäure, Terephthalsäure, Isophthalsäure, Agaricinsäure, Citronensäure, sowie deren C₁-C₁₀-Alkylester, Halogenide, und Anhydride,
· Diisocyanate, wie z.B. Toluylendiisocyanat, Bitoluylendiisocyanat, Dianisidindiisocyanat, Tetramethylendiisocyanat, Hexamethylendiisocyanat, m-Phenylendiisocyanat, m-Xylylendiisocyanat, C₁-C₆ Alkylbenzoldiisocyanat, 1-Chlorobenzol-2,4-diisocyanat, Cyclohexylmethandiisocyanat, 3,3'-Dimethoxydiphenylmethan-4,4'-diisocyanat, 1-Nitrobenzol-2,4diisocyanat, 1-Alkoxybenzol-2,4-diisocyanat, Ethylendiisocyanat, Propylendiisocyanat, Cyclohexylen-1,2-diisocyanat, 3,3'-Dichloro-4,4'-biphenylendiisocyanat, Diphenylendüsocyanat, 2-Chlorotrimethylendiisocyanat, Butylen-1,2-diisocyanat, Ethylidendiisocyanat, Diphenylmethan-4,4'diisocyanat, Diphenylethandiisocyanat, 1,5-Naphthalindiisocyanat, Cyclohexandiisocyanat und Isophorondiisocyanat,
· Diole, wie z.B. Ethylenglycol, propylenglycol, Butylenglycol, und Neopentylglycol, Pentandiol-1,5,3-Methylpentandiol-1,5, Bisphenol A, 1,2- oder 1,4-Cyclohexandiol, Caprolactondiol (Reaktionsprodukt aus Caprolacton und Ethylenglycol), hydroxyalkylierte Bisphenole, Trimethylolpropan, Trimethylolethan, Pentaerythritol, Hexandiol-1,6, Heptandiol-1,7, Octandiol-1,8, Butandiol-1,4, 2-Methyloctandiol-1,8, Nonandiol-1,9, Decandiol-1,10, Cyclohexandimethylol, Di-, Tri- und Tetraethylenglykol, Di-, Tri- und Tetrapropylenglykol, Polyethylen- und Polypropylenglykole mit einem mittleren Molekulargewicht von 150 bis 15.000, Trimethylolethan, Trimethylolpropan und Pentaerythrit,
· Diamine, wie z.B. 1,2-Diaminoethan, 1,2- oder 1,3-Diaminopropan, 1,2-, 1,3- oder 1,4-Diaminobutan, 1,5-Diaminopentan, 2,2-Dimethyl-1,3-diaminopropan, Hexamethylendiamin, 1,7-Diaminoheptan, 1,8-Diaminooctan, Trimethyl-1,6-diaminohexan, 1,9-Diaminononan, 1,10-Diaminodecan, 1,12-Diaminododecan, 1,2-Diaminocyclohexan, 1,4-Diaminocyclohexan, 1,3-Cyclohexan-bis(methylamin), 1,2-Phenylendiamin, 1,3-Phenylendiamin, 1,4-Phenylendiamin, 4,4'-Ethylendianilin, 4,4'-Methylendianilin, 4,4'-Diaminostilben, 4,4'-Thiodianilin, 4-Aminophenyldisulfid, 2,6-Diaminopyridin, 2,3-Diaminopyridin, 3,4-Diaminopyridin, 2,4-Diaminopyrimidin, 4,5-Diaminopyrimidin, 4,6-Diaminopyrimidin.

Die Verwendung solcher Linker-Moleküle ermöglicht die Kombination von Kolloiden X mit Wirkstoffen Z, die identische funktionelle Gruppen tragen, z.B. zweier Alkohole, und folglich nicht direkt unter Bildung eines Linkers L miteinander reagieren können.

Zur Durchführung des Verfahrens wird menschliches oder tierisches Blut nach der Entnahme mit einer wässrigen Zusammensetzung umfassend die Verbindung Mₙ-X-(L-Z)ₘ versetzt. Eine solche Zusammensetzung enthält die Verbindung Mₙ-X-(L-Z)ₘ in einer Konzentration von 0,0001 bis 50 Gew.-%, bevorzugt von 0,0005 bis 10 Gew.-%, besonders bevorzugt von 0,001 bis 1 Gew.-%, bezogen auf die Gesamtzusammensetzung. Die pharmazeutische Zusammensetzung ist direkt injizierbar.

Aufgrund der Eigenschaft von Makrophagen, Makromoleküle, insbesondere Kolloide, zu phagozytieren, werden die Kolloid-Arzneistoff-Komplexe Mₙ-X-(L-Z)ₘ von diesen aufgenommen. Gleichzeitig oder im Anschluss werden die Blutzellfraktionen in der Kolloid-Arzneistoff-Komplex-Lösung durch Zentrifugation aufgetrennt und abgehoben. Durch ein Dialyseverfahren werden danach die nicht phagozytierten Kolloid-Arzneistoff-Komplexe aus der Zellsuspension gewaschen. Da in den Kolloid-Anteil des Kolloid-Arzneistoff-Komplexes Fluoreszenz-Marker eingebaut sein können, kann das Maß der Aufnahme der Kolloid-Arzneistoff-Komplexe mittels Durchflusszytometrie quantifiziert werden. Schließlich kann die Zellsuspension infundiert werden. Die Zellen des Blutes sind ausgewählt aus der Gruppe der Makrophagen, Phagozyten, Monozyten, Histiozyten, Osteoklasten, Kupfer-Zellen, Granulozyten und Mikroglia-Zellen.

In einem Anwendungsbeispiel soll einem Patienten ein arzneilich wirksamer Stoff zur Behandlung einer Erkrankung im Bereich der lymphatischen Organe zugeführt werden. Für den arzneilich wirksamen Stoff soll dabei eine erhöhte Konzentration im Bereich der peripheren Lymphknoten erreicht werden. Hierfür wird dem Patienten Vollblut entnommen und mit der Arzneistoff-Kolloid-Verbindung in einem Zentrifugenröhrchen inkubiert. Um spezifische Interaktionen zwischen den immunkompetenten Zellen (nach Inkubation) in der Blutprobe zu verhindern, kann es notwendig werden, bestimmte Cluster dieser Zellen zu entfernen. Dies kann durch Adhäsion an geeignete Oberflächen erfolgen. Andere geeignete Verfahren sind die Gradientensedimentation. In vorteilhafter Ausgestaltung des Verfahrens wird das Patientenblut mit der Arzneistoff-Kolloid-Verbindung inkubiert und bei geeigneter Wahl von Molekulargewicht und chemischer Struktur des Kolloids (z. B. Dextran/Stärke) in diesem Medium zentrifugiert. Die Arzneistoff-Kolloid-Lösung dient dabei sowohl als Medium für die Gradientensedimentation, als auch Phagozytose-Substrat der Zellsuspension.

Es konnte festgestellt werden, dass durch dieses Verfahren eine schnelle und befriedigende Trennung der Zellen durch Zentrifugation erfolgen kann und gleichzeitig die Aufnahme der kolloidalen Makromoleküle in die Makrophagen erfolgt.

Je nachdem ob ein Einfluss auf die zellulären Heparin-Bindungsstellen für Chemokine gewünscht wird, kann die Vollblut-Arzneistoff-Suspension durch Heparin und/oder durch andere Verfahren, beispielsweise durch Zusatz von Natriumcitrat ungerinnbar gemacht werden. Danach wird das Blut durch entsprechende Dialyse und/oder Filtrationsverfahren von den nicht durch Phagozytose zellulär aufgenommenen Arzneistoff-Kolloid-Makromolekülen gereinigt.

Dementsprechend wird nach der Inkubation wird die Suspension bestehend aus Blut und der Arzneistoff-Kolloid-Lösung zentrifugiert. Hierdurch werden die roten von den weißen Blutkörperchen und verbliebenen Thrombozyten getrennt. Die Leukozyten befinden sich nach Zentrifugation in einer spezifischen Schicht des Gradientenmediums und können, im einfachsten Fall, beispielweise mit einer Pipette abgehoben werden. In einer anderen Ausgestaltung kann diese Zellschicht durch vorgefertigte Öffnungen in der Wand des Inkubations- und Zentrifugenröhrchens zum Abströmen gebracht und gesammelt werden. Danach wird die so separierte Leukozytenfraktion durch Dialyse von den nicht phagozytierten Arzneistoff-Kolloid-Molekülen befreit. Hierbei wird die Blut/Arzneistoff-Kolloid-Lösungssuspension von einer Dialyselösung durchströmt, die dabei eine Dialysemembran passiert. Dabei treten alle gelösten Bestandteile durch die Dialysemembran, die Leukozyten jedoch werden durch die Membran zurückgehalten und verbleiben in einem Rest der Dialyselösung, die danach aufgrund ihrer entsprechenden Zusammensetzung als Nähr- und Pufferlösung dient.

In vorteilhafter Ausgestaltung des Verfahrens kann die gewünschte Phagozytose der Arzneistoff-Kolloid-Komplexe beobachtet und quantifiziert werden. Eine Markierung des Kolloids durch einen Fluoreszenzmarker M, beispielsweise durch Fluoresceinisothiocyanat ermöglicht es, die Phagocytose des markierten Arzneistoff-Kolloid-Komplexes durch fluoreszenzmikroskopische Verfahren zu erfassen. Dies kann nach Entnahme aus einer abgeschlossenen Kontrollkammer geschehen. Diese Messungen sollten nach Reinigung von den nicht phagozytierten Arzneistoff-Kolloid-Komplexen in geeigneten Messkammern erfolgen. Abschließend kann das hergestellte Zellsuspensat dem Patienten infundiert werden.

Die Phagozytose-Erfassung und Quantifizierung kann aber auch kontinuierlich mittels Durchflusszytometrie in den entsprechend vorbereiteten Proben erfolgen. Bei Bedarf kann sogar die Durchflusszytometrie mit Zellsortern betrieben und die Zellen mit aufgenommenen fluoreszierendem Arzneistoff-Kolloid-Komplex separiert werden. Durch Sorterverfahren kann die Spezifität der injizierbaren Zellsuspensionen gesteigert werden, dieses Verfahren ist jedoch zeitaufwendig und damit mit einer niedrigeren Ausbeute an injizierbaren Zellen verbunden.

In einer weiteren Ausgestaltung betrifft die Erfindung ein Kits-of-Parts, umfassend die Fertiglösungen umfassend die erfindungsgemäße Verbindung I in wässriger Lösung, gegebenenfalls in Mehrkammersystemen umfassend Separations- und Dialysekammern und geeignete Mittel zur Regelung der Flüsse zwischen den Kammern.

In einer besonderen Ausgestaltung besteht ein solcher Kit-of-Parts aus
· mindestens einer Arzneistoff-Kolloid-Lösung;
· mindestens einem zur Zentrifugation geeigneten Gefäß;
· einer Pipetier oder Abflussvorrichtung für die separierte Zellfraktion;
· einem gerinnungshemmenden Stoff;
· einem Behälter für die Dialysat-Puffer-Lösung
· einer Dialyse-Kammer, die von einer Membran unterteilt ist, welche für die angewendeten Flüssigkeiten durchlässig ist, die Zellen jedoch zurückhält.

In einer weiteren besonderen Ausgestaltung sind alle Lösungen und Medien in einem Mehrkammersystem integriert, in welchem die Medien durch Kanäle und/oder Schläuche miteinander verbunden sind, deren Durchgängigkeit von außen gesteuert und kontrolliert werden kann. In der Wand des zur Phagozytose und Gradientensedimentation vorgesehenen Gefäßes befinden sich Auslassöffnungen und Kanäle, die durch Abdeckflächen verschlossen sind. Durch die Bewegung der Abdeckfläche kann ein Teil einer Auslassöffnung geöffnet werden. In einer alternativen Ausgestaltung befinden sich die Auslässe in einem inneren Gefäß, das durch Abdeckmittel eines äußeren Gefäßes verschlossen wird. Durch eine relative Verschiebung der beiden Gefäße gegeneinander kann eine mindestens teilweise Öffnung von einem der Auslässe erreicht werden.

Da die Zentrifugation ein wesentlicher Mechanismus der Zellseparation ist, ist der komplette Kit-of-Parts vorzugsweise geeignet, nach der Blutentnahme und Trennung vom Patienten in einen speziellen Zentrifugenrotor eingefügt zu werden. Durch diese Anordnung ist sichergestellt, dass das MehrkammerSystem lediglich bei der Inkubation mit Patientenblut sowie bei der Retransfusion (nach Phagozytose, Separation und Dialyse im geschlossenen System) mit der Außenwelt in Kontakt gebracht wird.

Die Phagozytose erfolgt hierbei in einem Röhrchen, das in die Zentrifuge eingesetzt werden kann. Das Röhrchen kann bereits während des Phagozytosevorgangs mit wählbaren und veränderbaren Drehzahlen und Temperaturen zentrifugiert werden. Lediglich am Ende der Gradientenseparation sollten die Leukozyten, insbesondere Monozyten, in einer spezifischen Zellschicht vorliegen. Diese Zellschicht kann einerseits bei einem Kit-of-Part durch eine vorinstallierte Pipetiervorrichtung abgehoben werden. Zum anderen ist es vorteilhaft, diese Zellen nach (während) der Zentrifugation durch mindestens einen kleinen Kanal in eine Dialysekammer abströmen zu lassen. Hier werden alle gelösten Bestandteile durch eine Dialysemembran geleitet, deren Ausschlussgröße so beschaffen ist, dass lediglich Zellen zurückgehalten werden. Die Zellsuspension kann anschließend in einem Rest der Dialyselösung verbleiben, die aufgrund ihrer entsprechenden Zusammensetzung auch als Nähr- und Pufferlösung für die Zellen dient. Durch den Einbau einer zur Fluoreszenzmikroskopie geeigneten Mess- und Zählkammer können die Zellen untersucht werden.

### Beispiele

### Beispiel 1

1 Mol Abbaustärke und 15 Mol Digoxin wurden durch Zugabe von NaOH auf einen pH von 8 bis 9 eingestellt. Unter intensivem Rühren wurden 25 Mol Malonsäuredichlorid langsam zugetropft. Nach wässriger Aufarbeitung wurden die Reaktionsprodukte durch Ultrafiltration (Membran 50.000 Dalton) von niedermolekularen Bestandteilen befreit und schließlich gefriergetrocknet.

### Beispiel 2

1 Mol einer Hydroxyethylstärke mit einem mittleren Substitutionsgrad DS von 0,3 und einem Molekulargewicht von 300.000 und 23 Mol einer Hydroxyethylstärke mit einem mittleren Substitutionsgrad DS von 0,6 und einem Molekulargewicht von 20.000 wurden unter Zugabe von NaOH auf einen pH von 8 bis 9 eingestellt. Unter intensivem Rühren wurden 25 Mol Malonsäuredichlorid langsam zugetropft. Nach wässriger Aufarbeitung wurden die Reaktionsprodukte durch Ultrafiltration (Membran 50.000 Dalton) von niedermolekularen Bestandteilen befreit und schließlich gefriergetrocknet

### Beispiel 3

15 Mol einer Hydroxyethylstärke mit einem mittleren Substitutionsgrad DS von 0,6 und einem Molekulargewicht von 15.000 wurden unter Zugabe von NaOH auf einen pH von 8 bis 9 eingestellt. Unter intensivem Rühren wurden 25 Mol Malonsäuredichlorid langsam zugetropft. Nach wässriger Aufarbeitung wurden die Reaktionsprodukte durch Ultrafiltration (Membran 75.000 Dalton) von niedermolekularen Bestandteilen befreit und schließlich gefriergetrocknet.

## Patentansprüche

1. Verbindung der allgemeinen Formel I
Mₙ-X-(L-Z)ₘ (I)
wobei
· X eine kolloidwirksame Verbindung,
· Z ein Arzneimittelwirkstoff,
· L einen Linker, durch den X mit Z kovalent verknüpft ist,
· M ein Fluoreszenzmarker,
· m eine ganze Zahl zwischen 1 und 10.000 ist und
· n 0 oder 1 ist.

2. Verbindung nach Anspruch 1, **dadurch gekennzeichnet, dass** der Linker L eine funktionelle Gruppe, ausgewählt aus Carbonsäureestern, Carbonsäureamiden und Urethanen ist.

3. Verbindung nach Anspruch 1 oder 2, erhältlich durch Reaktion eines Diamins der allgemeinen Formel II
R¹(-NH₂)₂ (II),
wobei R¹ ausgewählt ist aus
· einer Einfachbindung
· linearen oder verzweigten, gesättigten oder ungesättigten, aliphatischen oder alicyclischen Kohlenwasserstoffresten mit 1 bis 22 Kohlenstoffatomen;
· Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
· Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit einer freien funktionellen Gruppe des zugrundeliegenden Arzneimittelwirkstoffes Z und wenigstens einer freien funktionellen Gruppe des zugrundeliegenden Kolloids X, die jeweils unabhängig voneinander ausgewählt sind aus
· Carboxyl- (-COOH),
· Carbonsäurehalogenid- (-CO-A, mit A = Cl, Br oder I)
· Carboxylalkylen- (-(CH₂)_{q}-COOH, mit q = 1-10)
· oder Ester-Gruppen (-COOR),
unter Bildung des Linkers L.

4. Verbindung nach Anspruch 1 oder 2, erhältlich durch Reaktion eines Diols der allgemeinen Formel III
R¹(-OH)₂ (III),
wobei R¹ ausgewählt ist aus
· linearen oder verzweigten, gesättigten oder ungesättigten, aliphatischen oder alicyclischen Kohlenwasserstoffresten mit 2 bis 22 Kohlenstoffatomen;
· Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
· Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit einer freien funktionellen Gruppe des zugrundeliegenden Arzneimittelwirkstoffes Z und wenigstens einer freien funktionellen Gruppe des zugrundeliegenden Kolloids X, die jeweils unabhängig voneinander ausgewählt sind aus
· Carboxyl- (-COOH),
· Carbonsäurehalogenid- (-CO-A, mit A = Cl, Br oder I)
· Carboxylalkylen- (-(CH₂)_{q}-COOH, mit q = 1-10)
· Isocyanat- (-NCO)
· oder Ester-Gruppen (-COOR),
unter Bildung des Linkers L.

5. Verbindung nach Anspruch 1 oder 2, erhältlich durch Reaktion einer Dicarbonsäure der allgemeinen Formel IV
R¹(-COOH)₂ (IV)
wobei R¹ ausgewählt ist aus
· einer Einfachbindung
· linearen oder verzweigten, gesättigten oder ungesättigten, aliphatischen oder alicyclischen Kohlenwasserstoffresten mit 1 bis 22 Kohlenstoffatomen;
· Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
· Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit jeweils einer freien funktionellen Gruppe des zugrundeliegenden Arzneimittelwirkstoffes Z und wenigstens einer freien funktionellen Gruppe des zugrundeliegenden Kolloids X, die jeweils unabhängig voneinander ausgewählt sind aus
· Amino- (-NH₂),
· oder Hydroxyl-Gruppen (-OH),
unter Bildung des Linkers L.

6. Verbindung nach Anspruch 1 oder 2, erhältlich durch Reaktion eines Dicarbonsäurehalogenids der allgemeinen Formel V
R¹(-CO-A)₂, (V),
wobei A = Cl, Br oder I und R¹ ausgewählt ist aus
· einer Einfachbindung;
· linearen oder verzweigten, gesättigten oder ungesättigten, aliphatischen oder alicyclischen Kohlenwasserstoffresten mit 1 bis 22 Kohlenstoffatomen;
· Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
· Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit einer freien funktionellen Gruppe des zugrundeliegenden Arzneimittelwirkstoffes Z und wenigstens einer freien funktionellen Gruppe des zugrundeliegenden Kolloids X, die jeweils unabhängig voneinander ausgewählt sind aus
· Amino- (-NH₂),
· oder Hydroxyl-Gruppen (-OH),
unter Bildung des Linkers L.

7. Verbindung nach Anspruch 1 oder 2, erhältlich durch Reaktion einer Diesters der allgemeinen Formel VI
R¹(-COOR')₂ (VI)
wobei R' eine C₁₋₁₀-Alkylgruppe ist und R¹ ausgewählt ist aus
· einer Einfachbindung;
· linearen oder verzweigten, gesättigten oder ungesättigten, aliphatischen oder alicyclischen Kohlenwasserstoffresten mit 1 bis 22 Kohlenstoffatomen;
· Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
· Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit jeweils einer freien funktionellen Gruppe des zugrundeliegenden Arzneimittelwirkstoffes Z und wenigstens einer freien funktionellen Gruppe des zugrundeliegenden Kolloids X, die jeweils unabhängig voneinander ausgewählt sind aus
· Amino- (-NH₂),
· oder Hydroxyl-Gruppen (-OH),
unter Bildung des Linkers L.

8. Verbindung nach Anspruch 1 oder 2, erhältlich durch Reaktion eines Diisocyanats der allgemeinen Formel VII
R¹(-NCO)₂ (VII),
wobei R¹ ausgewählt ist aus
· inearen oder verzweigten, gesättigten oder ungesättigten, aliphatischen oder alicyclischen Kohlenwasserstoffresten mit 1 bis 22 Kohlenstoffatomen;
· Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
· Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit einer freien Hydroxyl-Gruppe (-OH) des zugrundeliegenden Arzneimittelwirkstoffes Z und wenigstens einer freien funktionellen Gruppe des zugrundeliegenden Kolloids X, unter Bildung des Linkers L.

9. Pharmazeutische wässrige Zusammensetzung umfassend die Verbindung Mₙ-X-(L-Z)ₘ nach irgendeinem der Ansprüche 1 bis 8.

10. Kit-of-parts umfassend räumlich getrennt voneinander die pharmazeutische Zusammensetzung nach Anspruch 9 sowie ein zur Zentrifugation geeignetes Gefäß.

11. Zellsuspension umfassend Zellen des Blutes, enthaltend die Verbindung Mₙ-X-(L-Z)ₘ, nach irgendeinem der Ansprüche 1 bis 8.

12. Verfahren zur Herstellung der Zellsuspenson wie in Anspruch 11 definiert durch
a. (ex-vivo) Inkubation von menschlichem oder tierischem Blut mit einer Verbindung Mₙ-X-(L-Z)ₘ, durch Vermischen der wässrigen Zusammensetzung, wie in Anspruch 9 definiert, mit dem Blut,
b. Abtrennung der inkubierten Blutzellen von der wässrigen Zusammensetzung gemäß Anspruch 9 durch Zentrifugation,
c. Befreiung der separierten inkubierten Blutzellen von nicht aufgenommener Verbindung Mₙ-X-(L-Z)ₘ durch Dialyse,
d. Isolierung der Zellsuspension, umfassend die inkubierten Blutzellen.

13. Verfahren nach Anspruch 12, **dadurch gekennzeichnet, dass** die inkubierten Blutzellen Leukozyten sind.

14. Verfahren zur Herstellung der Zellsuspension wie in Anspruch 11 definiert, **dadurch gekennzeichnet, dass** eine Quantifizierung und/oder Selektierung der phagocytierten Verbindung Mₙ-X-(L-Z)ₘ für n = 1 mittels Fluoreszenzmikroskopie oder Durchflusscytometrie erfolgt.

15. Verwendung der Zellsuspension nach Anspruch 11 zur Herstellung eines Medikaments zur gezielten Aufnahme eines Wirkstoffes Z in spezifische Organe des menschlichen oder tierischen Körpers.

## Claims

1. A compound of general formula I
Mₙ-X-(L-Z)ₘ (I),
wherein
- X is a colloid-effective compound;
- Z is a medicinal drug;
- L is a linker through which X is covalently linked with Z;
- M is a fluorescent marker;
- m is an integer of from 1 to 10,000; and
- n is 0 or 1.

2. The compound according to claim 1, **characterized in that** said linker L is a functional group selected from carboxylic acid esters, carboxylic acid amides and urethanes.

3. The compound according to claim 1 or 2, obtainable by reacting a diamine of general formula II
R¹(NH₂)₂ (II)
wherein R¹ is selected from
- a single bond;
- linear or branched, saturated or unsaturated, aliphatic or alicyclic hydrocarbon residues with 1 to 22 carbon atoms;
- aryl, aryl-C₁-C₄-alkyl and aryl-C₂-C₆-alkenyl groups with 5 to 12 carbon atoms in the aryl residue which may optionally be substituted with C₁-C₆ alkyl and/or C₂-C₆ alkoxy groups; or
- heteroaryl, heteroaryl-C₁-C₄-alkyl and heteroaryl-C₂-C₆-alkenyl groups with 3 to 8 carbon atoms in the heteroaryl residue and one or two heteroatoms selected from N, O and S which may be substituted with C₁-C₆ alkyl and/or C₂-C₆ alkoxy groups;
with a free functional group of the underlying medicinal drug Z and at least one free functional group of the underlying colloid X, which are independently selected from
- carboxy (-COOH);
- carboxylic acid halide (-CO-A, with A = Cl, Br or I);
- carboxyalkylene (-(CH₂)_{q}-COOH, with q = 1-10); or
- ester groups (-COOR);
to form the linker L.

4. The compound according to claim 1 or 2, obtainable by reacting a diol of general formula III
R¹(OH)₂ (III),
wherein R¹ is selected from
- linear or branched, saturated or unsaturated, aliphatic or alicyclic hydrocarbon residues with 2 to 22 carbon atoms;
- aryl, aryl-C₁-C₄-alkyl and aryl-C₂-C₆-alkenyl groups with 5 to 12 carbon atoms in the aryl residue which may optionally be substituted with C₁-C₆ alkyl and/or C₂-C₆ alkoxy groups; or
- heteroaryl, heteroaryl-C₁-C₄-alkyl and heteroaryl-C₂-C₆-alkenyl groups with 3 to 8 carbon atoms in the heteroaryl residue and one or two heteroatoms selected from N, O and S which may be substituted with C₁-C₆ alkyl and/or C₂-C₆ alkoxy groups;
with a free functional group of the underlying medicinal drug Z and at least one free functional group of the underlying colloid X, which are independently selected from
- carboxy (-COOH);
- carboxylic acid halide (-CO-A, with A = Cl, Br or I);
- carboxyalkylene (-(CH₂)_{q}-COOH, with q = 1-10);
- isocyanate (-NCO); or
- ester groups (-COOR);
to form the linker L.

5. The compound according to claim 1 or 2, obtainable by reacting a dicarboxylic acid of general formula IV
R¹(COOH)₂ (IV),
wherein R¹ is selected from
- a single bond;
- linear or branched, saturated or unsaturated, aliphatic or alicyclic hydrocarbon residues with 1 to 22 carbon atoms;
- aryl, aryl-C₁-C₄-alkyl and aryl-C₂-C₆-alkenyl groups with 5 to 12 carbon atoms in the aryl residue which may optionally be substituted with C₁-C₆ alkyl and/or C₂-C₆ alkoxy groups; or
- heteroaryl, heteroaryl-C₁-C₄-alkyl and heteroaryl-C₂-C₆-alkenyl groups with 3 to 8 carbon atoms in the heteroaryl residue and one or two heteroatoms selected from N, O and S which may be substituted with C₁-C₆ alkyl and/or C₂-C₆ alkoxy groups;
respectively with a free functional group of the underlying medicinal drug Z and at least one free functional group of the underlying colloid X, which are independently selected from
- amino (-NH₂); or
- hydroxy groups (-OH);
to form the linker L.

6. The compound according to claim 1 or 2, obtainable by reacting a dicarboxylic acid halide of general formula V
R¹(CO-A)₂ (V),
wherein A = Cl, Br or I, and R¹ is selected from
- a single bond;
- linear or branched, saturated or unsaturated, aliphatic or alicyclic hydrocarbon residues with 1 to 22 carbon atoms;
- aryl, aryl-C₁-C₄-alkyl and aryl-C₂-C₆-alkenyl groups with 5 to 12 carbon atoms in the aryl residue which may optionally be substituted with C₁-C₆ alkyl and/or C₂-C₆ alkoxy groups; or
- heteroaryl, heteroaryl-C₁-C₄-alkyl and heteroaryl-C₂-C₆-alkenyl groups with 3 to 8 carbon atoms in the heteroaryl residue and one or two heteroatoms selected from N, O and S which may be substituted with C₁-C₆ alkyl and/or C₂-C₆ alkoxy groups;
with a free functional group of the underlying medicinal drug Z and at least one free functional group of the underlying colloid X, which are independently selected from
- amino (-NH₂); or
- hydroxy groups (-OH);
to form the linker L.

7. The compound according to claim 1 or 2, obtainable by reacting a diester of general formula VI
R¹(COOR')₂ (VI),
wherein R' is a C₁₋₁₀ alkyl group, and R¹ is selected from
- a single bond;
- linear or branched, saturated or unsaturated, aliphatic or alicyclic hydrocarbon residues with 1 to 22 carbon atoms;
- aryl, aryl-C₁-C₄-alkyl and aryl-C₂-C₆-alkenyl groups with 5 to 12 carbon atoms in the aryl residue which may optionally be substituted with C₁-C₆ alkyl and/or C₂-C₆ alkoxy groups; or
- heteroaryl, heteroaryl-C₁-C₄-alkyl and heteroaryl-C₂-C₆-alkenyl groups with 3 to 8 carbon atoms in the heteroaryl residue and one or two heteroatoms selected from N, O and S which may be substituted with C₁-C₆ alkyl and/or C₂-C₆ alkoxy groups;
respectively with a free functional group of the underlying medicinal drug Z and at least one free functional group of the underlying colloid X, which are independently selected from
- amino (-NH₂); or
- hydroxy groups (-OH);
to form the linker L.

8. The compound according to claim 1 or 2, obtainable by reacting a diisocyanate of general formula VII
R¹(NCO)₂ (VII),
wherein R¹ is selected from
- linear or branched, saturated or unsaturated, aliphatic or alicyclic hydrocarbon residues with 1 to 22 carbon atoms;
- aryl, aryl-C₁-C₄-alkyl and aryl-C₂-C₆-alkenyl groups with 5 to 12 carbon atoms in the aryl residue which may optionally be substituted with C₁-C₆ alkyl and/or C₂-C₆ alkoxy groups; or
- heteroaryl, heteroaryl-C₁-C₄-alkyl and heteroaryl-C₂-C₆-alkenyl groups with 3 to 8 carbon atoms in the heteroaryl residue and one or two heteroatoms selected from N, O and S which may be substituted with C₁-C₆ alkyl and/or C₂-C₆ alkoxy groups;
with a free hydroxy group (-OH) of the underlying medicinal drug Z and at least one free functional group of the underlying colloid X to form the linker L.

9. A pharmaceutical aqueous composition comprising the compound Mₙ-X-(L-Z)ₘ according to any of claims 1 to 8.

10. A kit of parts, comprising, separated from each other in space, the pharmaceutical composition according to claim 9 and a vessel suitable for centrifugation.

11. A cell suspension comprising cells of blood, containing the compound Mₙ-X-(L-Z)ₘ according to any of claims 1 to 8.

12. A method for preparing the cell suspension as defined in claim 11 by
a. (ex vivo) incubation of human or animal blood with a compound Mₙ-X-(L-Z)ₘ by mixing the aqueous composition as defined in claim 9 with the blood;
b. separating the incubated blood cells from the aqueous composition according to claim 9 by centrifugation;
c. freeing the separated incubated blood cells from non-absorbed compound Mₙ-X-(L-Z)ₘ by dialysis;
d. isolating the cell suspension comprising the incubated blood cells.

13. The method according to claim 12, **characterized in that** said incubated blood cells are leukocytes.

14. A method for preparing the cell suspension as defined in claim 11, **characterized in that** a quantification and/or selection of the phagocytosed compound Mₙ-X-(L-Z)ₘ is effected for n = 1 by means of fluorescence microscopy and flow cytometry.

15. Use of the cell suspension according to claim 11 for preparing a medicament for the selective uptake of a drug Z in specific organs of human or animal bodies.

## Revendications

1. Composé de formule générale I
Mₙ-X-(L-Z)ₘ (I)
où
- X est un composé efficace du point de vue colloïdal,
- Z est un principe actif médicamenteux,
- L est un lieur par lequel X est lié à Z de manière covalente,
- M est un marqueur de fluorescence,
- m est un nombre entier entre 1 et 10000 et
- n est 0 ou 1.

2. Composé selon la revendication 1 **caractérisé en ce que** le lieur L est un groupe fonctionnel choisi parmi les esters d'acides carboxyliques, les amides d'acides carboxyliques et les uréthanes.

3. Composé selon les revendications 1 ou 2 pouvant être obtenu par réaction d'une diamine de formule générale II
R¹(-NH₂)₂ (II)
où R¹ est choisi parmi
- une simple liaison ;
- les groupements hydrocarbonés aliphatiques ou alicycliques, saturés ou insaturés, linéaires ou ramifiés, ayant 1 à 22 atomes de carbone ;
- les groupes aryle, aryl-C₁-C₄-alkyle et aryl-C₂-C₆-alcényle ayant 5 à 12 atomes de carbone dans le groupement aryle, qui peuvent éventuellement être substitués par des groupes C₁-C₆-alkyle et/ou C₂-C₆-alcoxy ;
ou
- les groupes hétéroaryle, hétéroaryl-C₁-C₄-akyle et hétéroaryl-C₂-C₆-alcényle ayant 3 à 8 atomes de carbone dans le groupement hétéroaryle et un ou deux hétéroatomes choisis parmi N, O et S, qui peuvent être substitués par des groupes C₁-C₆-alkyle et/ou C₂-C₆-alcoxy ;
avec un groupe fonctionnel libre du principe actif médicamenteux de base Z et au moins un groupe fonctionnel libre du colloïde de base X, qui sont choisis dans chaque cas indépendamment l'un de l'autre parmi les groupes
- carboxyle (-COOH),
- halogénure d'acide carboxylique (-CO-A, avec A = Cl, Br ou I),
- carboxylalkylène (-(CH₂)_{q}-COOH, avec q = 1-10),
- ou ester (-COOR),
avec formation du lieur L.

4. Composé selon les revendications 1 ou 2 pouvant être obtenu par réaction d'un diol de formule générale III
R¹(-OH)₂ (III)
où R¹ est choisi parmi
- les groupements hydrocarbonés aliphatiques ou alicycliques, saturés ou insaturés, linéaires ou ramifiés, ayant 2 à 22 atomes de carbone ;
- les groupes aryle, aryl-C₁-C₄-alkyle et aryl-C₂-C₆-alcényle ayant 5 à 12 atomes de carbone dans le groupement aryle, qui peuvent éventuellement être substitués par des groupes C₁-C₆-alkyle et/ou C₂-C₆-alcoxy ;
ou
- les groupes hétéroaryle, hétéroaryl-C₁-C₄-alkyle et hétéroaryl-C₂-C₆-alcényle ayant 3 à 8 atomes de carbone dans le groupement hétéroaryle et un ou deux hétéroatomes choisis parmi N, O et S, qui peuvent être substitués par des groupes C₁-C₆-alkyle et/ou C₂-C₆-alcoxy ;
avec un groupe fonctionnel libre du principe actif médicamenteux de base Z et au moins un groupe fonctionnel libre du colloïde de base X, qui sont choisis dans chaque cas indépendamment l'un de l'autre parmi les groupes
- carboxyle (-COOH),
- halogénure d'acide carboxylique (-CO-A, avec A = Cl, Br ou I),
- carboxylalkylène (-(CH₂)_{q}-COOH, avec q = 1-10),
- isocyanate (-NCO),
- ou ester (-COOR),
avec formation du lieur L.

5. Composé selon les revendications 1 ou 2 pouvant être obtenu par réaction d'un acide dicarboxylique de formule générale IV
R¹(-COOH)₂ (IV)
où R¹ est choisi parmi
- une simple liaison ;
- les groupements hydrocarbonés aliphatiques ou alicycliques, saturés ou insaturés, linéaires ou ramifiés, ayant 1 à 22 atomes de carbone ;
- les groupes aryle, aryl-C₁-C₄-alkyle et aryl-C₂-C₆-alcényle ayant 5 à 12 atomes de carbone dans le groupement aryle, qui peuvent éventuellement être substitués par des groupes C₁-C₆-alkyle et/ou C₂-C₆-alcoxy ;
ou
- les groupes hétéroaryle, hétéroaryl-C₁-C₄-alkyle et hétéroaryl-C₂-C₆-alcényle ayant 3 à 8 atomes de carbone dans le groupement hétéroaryle et un ou deux hétéroatomes choisis parmi N, O et S, qui peuvent être substitués par des groupes C₁-C₆-alkyle et/ou C₂-C₆-alcoxy ;
avec un groupe fonctionnel libre du principe actif médicamenteux de base Z et au moins un groupe fonctionnel libre du colloïde de base X, qui sont choisis dans chaque cas indépendamment l'un de l'autre parmi les groupes
- amino (-NH₂),
- ou hydroxyle (-OH),
avec formation du lieur L.

6. Composé selon les revendications 1 ou 2 pouvant être obtenu par réaction d'un halogénure d'acide dicarboxylique de formule générale V
R¹(-CO-A)₂ (V)
où A = Cl, Br ou I et R¹ est choisi parmi
- une simple liaison ;
- les groupements hydrocarbonés aliphatiques ou alicycliques, saturés ou insaturés, linéaires ou ramifiés, ayant 1 à 22 atomes de carbone ;
- les groupes aryle, aryl-C₁-C₄-alkyle et aryl-C₂-C₆-alcényle ayant 5 à 12 atomes de carbone dans le groupement aryle, qui peuvent éventuellement être substitués par des groupes C₁-C₆-alkyle et/ou C₂-C₆-alcoxy ;
ou
- les groupes hétéroaryle, hétéroaryl-C₁-C₄-alkyle et hétéroaryl-C₂-C₆-alcényle ayant 3 à 8 atomes de carbone dans le groupement hétéroaryle et un ou deux hétéroatomes choisis parmi N, O et S, qui peuvent être substitués par des groupes C₁-C₆-alkyle et/ou C₂-C₆-alcoxy ;
avec un groupe fonctionnel libre du principe actif médicamenteux de base Z et au moins un groupe fonctionnel libre du colloïde de base X, qui sont choisis dans chaque cas indépendamment l'un de l'autre parmi les groupes
- amino (-NH₂),
- ou hydroxyle (-OH),
avec formation du lieur L.

7. Composé selon les revendications 1 ou 2 pouvant être obtenu par réaction d'un diester de formule générale VI
R¹(-COOR')₂ (VI)
où R' est un groupe C₁₋₁₀-alkyle et R¹ est choisi parmi
- une simple liaison ;
- les groupements hydrocarbonés aliphatiques ou alicycliques, saturés ou insaturés, linéaires ou ramifiés, ayant 1 à 22 atomes de carbone ;
- les groupes aryle, aryl-C₁-C₄-alkyle et aryl-C₂-C₆-alcényle ayant 5 à 12 atomes de carbone dans le groupement aryle, qui peuvent éventuellement être substitués par des groupes C₁-C₆-alkyle et/ou C₂-C₆-alcoxy ;
ou
- les groupes hétéroaryle, hétéroaryl-C₁-C₄-alkyle et hétéroaryl-C₂-C₆-alcényle ayant 3 à 8 atomes de carbone dans le groupement hétéroaryle et un ou deux hétéroatomes choisis parmi N, O et S, qui peuvent être substitués par des groupes C₁-C₆-alkyle et/ou C₂-C₆-alcoxy ;
avec un groupe fonctionnel libre du principe actif médicamenteux de base Z et au moins un groupe fonctionnel libre du colloïde de base X, qui sont choisis dans chaque cas indépendamment l'un de l'autre parmi les groupes
- amino (-NH₂),
- ou hydroxyle (-OH),
avec formation du lieur L.

8. Composé selon les revendications 1 ou 2 pouvant être obtenu par réaction d'un diisocyanate de formule générale VII
R¹(-NCO)₂ (VII)
où R¹ est choisi parmi
- les groupements hydrocarbonés aliphatiques ou alicycliques, saturés ou insaturés, linéaires ou ramifiés, ayant 1 à 22 atomes de carbone ;
- les groupes aryle, aryl-C₁-C₄-alkyle et aryl-C₂-C₆-alcényle ayant 5 à 12 atomes de carbone dans le groupement aryle, qui peuvent éventuellement être substitués par des groupes C₁-C₆-alkyle et/ou C₂-C₆-alcoxy ;
ou
- les groupes hétéroaryle, hétéroaryl-C₁-C₄-alkyle et hétéroaryl-C₂-C₆-alcényle ayant 3 à 8 atomes de carbone dans le groupement hétéroaryle et un ou deux hétéroatomes choisis parmi N, O et S, qui peuvent être substitués par des groupes C₁-C₆-alkyle et/ou C₂-C₆-alcoxy ;
avec un groupe hydroxyle (-OH) libre du principe actif médicamenteux de base Z et au moins un groupe fonctionnel libre du colloïde de base X, avec formation du lieur L.

9. Composition aqueuse pharmaceutique comprenant le composé Mₙ-X-(L-Z)ₘ selon l'une quelconque des revendications 1 à 8.

10. Kit-of-parts comprenant, séparés spatialement l'un de l'autre, la composition pharmaceutique selon la revendication 9 ainsi qu'un récipient approprié pour la centrifugation.

11. Suspension de cellules comprenant des cellules du sang, contenant le composé Mₙ-X-(L-Z)ₘ selon l'une quelconque des revendications 1 à 8.

12. Procédé de production de la suspension de cellules telle que définie dans la revendication 11 par
a. incubation (ex vivo) de sang humain ou animal avec un composé Mₙ-X-(L-Z)ₘ par mélange de la composition aqueuse telle que définie dans la revendication 9, avec le sang,
b. séparation des cellules sanguines incubées d'avec la composition aqueuse, selon la revendication 9, par centrifugation,
c. libération des cellules sanguines incubées séparées du composé Mₙ-X-(L-Z)ₘ non absorbé par dialyse,
d. isolement de la suspension de cellules comprenant les cellules sanguines incubées.

13. Procédé selon la revendication 12 **caractérisé en ce que** les cellules sanguines incubées sont des leucocytes.

14. Procédé de production de la suspension de cellules telle que définie dans la revendication 11 **caractérisé en ce que** l'on réalise une quantification et/ou une sélection du composé Mₙ-X-(L-Z)ₘ phagocyté pour n = 1 par microscopie à fluorescence ou cytométrie de flux.

15. Utilisation de la suspension de cellules selon la revendication 11 pour la production d'un médicament pour l'absorption ciblée d'un principe actif Z dans des organes spécifiques du corps humain ou animal.
